# EUROPEAN PATENT SPECIFICATION

(11) **EP 1 810 624 B1**
(45) Date of publication and mention of the grant of the patent: **18.07.2012**
(21) Application number: 07101017.7
(22) Date of filing: 23.01.2007
(51) Int. Cl.: A61B 17/70

(54) **Connecting rod with external adjustment element**
Verbindungsstab mit externem Verstellelement
Connexion d'une tige avec un élément de réglage externe

(30) Priority: 24.01.2006 DE 102006003374
(43) Date of publication of application: 25.07.2007
(73) Proprietor: BIEDERMANN MOTECH GmbH, 78054 VS-Schwenningen (DE)
(72) Inventor: Biedermann, Lutz, 78048, VS-Villingen (DE); Matthis, Wilfried, 79367, Weisweil (DE); Harms, Jürgen, 76227, Karlsruhe (DE); Carpenter, Scott, Fremont, CA 94539 (US); Dinh, Minh, Fremont, CA 94539 (US); Basude, R., Fremont, CA 94539 (US)
(74) Representative: Lang, Christian

(56) References cited:
- EP-A1- 1 574 173
- WO-A-2004/105577
- WO-A-2005/092222
- WO-A-2005/094704
- US-A- 5 672 175
- US-A1- 2005 171 540
- US-A1- 2005 288 672

## Description

### BACKGROUND

### FIELD OF THE INVENTION

The present invention relates generally to a flexible connecting element and to a flexible stabilization system with such a flexible connecting element, especially for use in spinal surgery. Furthermore, the invention also relates to a corresponding modular system.

### PRIOR ART

Flexible stabilization devices for the spine are known. US 2003/0109880 A1 discloses a stabilization system with an elastic rod element, which is provided as a connecting element between two bone anchors, whereby the elastic rod element is formed from a helical spring with narrow threads.

WO 2005/030031 A2 and WO 2005/039454 A2 similarly disclose flexible spinal stabilization systems in which different types of flexible connecting rods are provided between bone anchors. These patent documents propose that flexible connecting rods for achieving flexibility or mobility should have spiral cuts or slits and be combined with core rods in order that both, flexibility and rigidity, or together defined as spring properties, may be adjusted correspondingly.

Similar considerations apply to EP 0 677 277 A2, which also describes a dynamic or flexible stabilization device, especially for the spine, in which, again, a rod with cuts or a spiral slit is provided as a flexible connecting element. A viscoelastic material may be filled into the cavity of the part with the slit.

WO 03/047442 A1, in turn, proposes a damping element and a device for stabilizing adjacent vertebral bodies. According to the solution proposed in WO 03/047442, in a first embodiment two elastic elements coaxially arranged inside each other and consisting of a spirally slit tube and a core element are connected to two connecting elements. In a second embodiment, a tubular spring element with a spiral arrangement of slits has a one-piece connector and a receiving opening for adjacent flexible elements, with a second spring element provided as core element within the first spring element.

US 2003/0220643 A1 similarly discloses a flexible stabilization device for the spine, whereby, again, spiral-spring-like connecting rods are provided between pedicle screws or bone anchoring elements. US 2003/0220643 A1 additionally proposes providing the spiral-spring-like connecting rods with sleeves which act as spacers between the pedicle screws to prevent too much deformation or mobility.

WO 2004/105577 A2, in turn, describes a spinal stabilization system in which differently shaped, flexible connecting rods are used between the bone anchors. They consist of metal strips wound spirally into tubes or of structures braided from metal fibers. Also described are spring elements which are inserted between rods or spirally slit cylindrical tubes with and without cores. Further connecting elements are disclosed in documents WO 2005/092 222 A, EP-A1-1 574 173 and US 2005/171 540 A1.

### DISCLOSURE OF THE INVENTION

### OBJECT OF THE INVENTION

Although some of the described prior-art stabilization systems already exhibit very good properties, the goal remains to create a stabilization device, and especially a flexible connecting element for arrangement between bone and vertebral anchors, that replicates the complex, mechanical properties of the spine to the best possible extent such that adequate support combined with simultaneous retention of mobility may be provided in damaged spinal regions. Such a stabilization system, optimally adapted to provide the complex functions above, should be easy to manufacture and also easy to manipulate during use, i.e., by the surgeon during surgery.

### TECHNICAL SOLUTION

This object is achieved with a flexible connecting element having the features of claim 1, a stabilization system with the features of claim 25 and a modular system from the respective components in accordance with the features of claim 27. Advantageous embodiments are the object of the dependent claims.

According to a first aspect, the present invention is a flexible connecting element for implantation into the human or animal body, especially as a connecting element or connecting rod for stabilizing the spine. The flexible connecting element of the invention comprises a first rod that has at least one flexible section and at least two connecting sections, the latter of which are adapted for attachment to anchoring devices to be secured to the human or animal body, especially anchoring elements for the vertebrae of the spine. Such a flexible connecting element facilitates stabilization by load accommodation combined with simultaneous mobility via the flexible section. Thus far, a flexible connecting element as described above corresponds to known flexible connecting rods, for example for use in stabilization systems for the spine.

However, the flexible connecting element of the invention features, in addition to the above-described first rod, a property adjustment element, which extends along at least a part of the flexible section of the first rod. More precisely, the property adjustment element can be a flexible element or a stabilization element located outside the rod cross-section, with the flexible element or the stabilization element forming an outer element connected to the first rod in at least one location such that it cannot be displaced axially.

In the following, the property adjustment element is described as a flexible element or a stabilization element. The flexible element is defined as an element primarily providing flexibility properties. However, aside from having a certain flexibility and thus mobility, and especially reversible deformability, a flexible outer element can additionally take on stabilization functions, which are inherent in the design. For example, the flexible outer element arranged outside the rod cross-section may provide stabilization with respect to specific loads, for example shearing loads, while other loads like bending forces lead to a deformation of the flexible outer element. Thus, a flexible element being primarily flexible is not restricted to be merely flexible, but can provide stabilization in specific load cases.

In the understanding of the present patent, a stabilization element is accordingly defined by the primary function of stabilization, even if a small degree of inherent flexibility or elasticity properties may be present in the stabilization element. Thus, the stabilization element is primarily stiff and rigid, although a minor degree of flexibility may be observed under certain loads. However, the stabilization element may facilitate a certain degree of reversible deformability or mobility of the first rod in order to effectuate the flexible or dynamic properties of the connecting element of the invention without being deformed itself. This can occur in such a manner that, in certain directions of movement or in certain forms of movement, the stabilization element provides the first rod with sufficient scope of movement without itself having to be deformed or moved. For example, due to the connection of the stabilization element arranged outside the rod cross-section to the first rod in only one connecting location, the first rod may for example be compressed in an axial direction or bended about axes perpendicular to the longitudinal axis, while the essentially stiff outer element is neither loaded nor deformed during the motion of the first rod. Thus, the design of the outer element is such that the first rod can perform specific movements without affecting the outer element. However, other movements cannot be carried out, since the outer element is designed such that this is prevented, for example deformation or movement due to shearing loads.

The arrangement of the flexible element or stabilization element outside the rod cross-section facilitates additional adjustment of the mechanical properties of the flexible connecting element in addition to an adjustment via the shape of the first or primary rod and a core provided optionally therein. Thus, the flexible connecting element of the invention is suitable for use in a modular system in which the surgeon can make an adjustment to the mechanical properties of the flexible connecting element by replacing individual components, namely the flexible element or stabilization element. The modular concept allows for easy manufacturing and moreover facilitates simplified and smaller inventory for industry (manufacturers, sales) and clinics.

Moreover, through the external arrangement, the connecting technique between flexible element or stabilization element on the one hand and the first rod on the other hand is markedly simplified. Further, deliberate arrangement of the flexible element or stabilization element solely in the flexible section of the first rod or a part thereof can allow particularly accurate adjustment of the mechanical properties of the flexible connecting element, such as reversible deformability, extensibility, etc.

The flexible element or stabilization element may be formed as a rod, strip, partial cylinder, especially a half-shell or similar, or as a cylindrical-tubular body, as described in further detail below. These simple geometrical forms also facilitate simple connection between the flexible element or stabilization element on the one hand and the first rod on the other.

Similar to the flexible section of the first rod, the flexible element can achieve its flexibility or reversible deformability both by design, such as cross-sectional and/or wall thickness reductions, and/or the provision of material recesses and/or by the choice of a correspondingly flexible material. Consequently, various possibilities for achieving flexibility or reversible deformability are available for the various applications and areas of application.

Preferred, however, is the formation of the flexible element as a cylindrical-tubular body that has at least one slit and/or grooved material recess, which is especially provided spirally or helically about the longitudinal axis of the cylindrical-tubular body. The slit and/or grooved material recess can pass completely through the wall thickness of the cylindrical-tubular body (slit) or a residual wall thickness may remain (groove).

Through such a design, the possibility of axial extension and/or compression, torsion about the longitudinal axis, and/or bending about a radial axis (transverse axis) is created, such that all movements are possible in all spatial directions. Correspondingly, it is also preferred that the flexible section of the first rod is formed in a similar way.

It is advantageous, if the flexible element or stabilization element is provided exclusively in the flexible section(s) of the first rod, since only these sections require corresponding adjustment of the mechanical properties via the flexible element or stabilization element.

This also makes it possible for the flexible element or the stabilization element to be connected to the first rod in the section between the connecting section and the flexible section of the first rod for which purpose preferably positive and/or non-positive joints are provided and/or frictional connections and/or material connections.

The positive and/or non-positive joints can, for example, be formed by catches, such as hooks or projecting noses that engage with openings, pins that also engage with corresponding openings, or screw connections with screws in threaded holes.

A preferred embodiment of catches consists of, for example, at the flexible element or stabilization element, an inwardly projecting protuberance that engages with a recess of the first rod, said protuberance especially being arranged on a spring-loaded flap or bending flap.

Thus, in a simple but effective manner, a connection can be achieved between the flexible element or stabilization element on the one hand and the first rod on the other, said connection avoiding or ruling out an axial displacement of the flexible element or stabilization element relative to the first rod at the connection in the direction of the longitudinal axis of the first rod.

Preferably, only a single connecting location, especially in a plane perpendicular to the longitudinal axis, is provided between the flexible element or the stabilization element on the one hand and the first rod on the other. This makes it possible for the first rod to extend in the axial direction independently of the flexible element or stabilization element. Correspondingly, the flexibility or reversible mobility or deformability of the first rod in the axial direction is not influenced by the flexible element or stabilization element and the adjustment of the mechanical properties or a stabilization function becomes effective only as regards the remaining types of deformation or movement.

The first rod may preferably be formed from a cylindrical solid body or cylindrical-tubular body in which preferably additional core elements in the form of one or several second rods may be provided that may be arranged coaxially with each other.

The second rod may, in the same manner as the first rod, have one or more flexible sections or be formed such that overall it is flexible or reversibly deformable.

The second rod may preferably, like the flexible element or the stabilization element, be connected to the first rod at just one location, such that the rods may be deformed or moved independently of each other in the axial direction. Therefore, the connection between the first rod and the second rod(s) is preferably provided in the end section of the rods.

The first and/or second rod may, as already mentioned, be shaped with its flexible section similar to that of the flexible element. However, in order that an additional stabilization function may be obtained, especially with regard to transverse forces or flexural stress, the threads of the spiral slit and/or grooved material recesses of adjacent components, namely flexible element and first rod or first rod and second rod may for example be formed such that the windings and threads run in opposite directions or are mirror-symmetrical.

In addition, the helical or spiral shapes of the flexible section or the flexible element may be characterised by the fact that, for example, a double helix is used and that the radius or diameter is enlarged or reduced, such that the spiral section has the shape of a waist, stomach or barrel.

The first rod, a second or third rod, or other additional rods and the flexible element may be made of a flexible material, especially a bio-compatible plastic or metal. Preferred is the use of a super- or pseudo-elastic material and here especially the use of nitinol, a nickel-titanium alloy. A material of this kind possesses the special properties that are advantageous for the aforementioned application.

Candidate plastics are especially polyethylene and polytetrafluoroethylene (Teflon), as well as polyethylene terephthalate (PET).

Preferably, the first rod and/or the outer element are made of nitinol and a second or other core rods of plastic.

To keep ease of manufacture as simple as possible and also to facilitate convenience during surgery, it has proved successful to form the flexible element, the first rod, the second rod and/or any additional rods, each as one piece.

According to a second aspect of the present invention, a connecting element of the invention is used in a flexible stabilization system, especially for the spine, in which the flexible connecting element serves to connect bone anchors, so-called anchoring elements, which are attachable to vertebrae of the spine. Both single-segment and multi-segment implantation are possible. A segment is meant here to be two adj acent vertebrae with an intervertebral disc arranged between them. A multi-segment implantation therefore means the connection of more than two vertebral bodies to the flexible connecting element.

The anchoring elements can be pedicle screws, which may be formed as polyaxial screws in order that arbitrary alignment of the connecting element to the anchoring screw may be ensured.

According to a third aspect of the present invention, not only the stabilization system but also the flexible connecting element is formed as a modular system, which enables the surgeon to adjust the implant to the patient concerned during the intervention, by, for example, exchanging the flexible element or the stabilization element of the flexible connecting element.

### BRIEF DESCRIPTION OF THE FIGURES

Further advantages, characteristics and features of the present invention are apparent from the following detailed description of embodiments using the enclosed drawings, which are shown in purely schematic form.

Fig. 1 shows a three-dimensional representation of a connecting element of the invention;

Fig. 2 shows an enlarged representation of the section from Fig. 1;

Fig. 3 shows a three-dimensional representation of the connecting element from Figs. 1 and 2 in the disassembled state;

Fig. 4 shows a three-dimensional detailed view of the flexible element of connecting element from Fig. 1;

Fig. 5 shows a three-dimensional detailed view of the first rod of connecting element from Fig. 1;

Fig. 6 shows a three-dimensional representation of an alternative connection between flexible element and the first rod of a connecting element of the invention;

Figs. 7a and 7b show two three-dimensional representations a) and b) of stabilization systems of the invention.

Fig. 8 shows a three-dimensional representation of an alternative embodiment of the connecting element of the invention;

Fig. 9 shows a three-dimensional representation of another alternative connecting element of the invention; and

Fig. 10 shows a three-dimensional representation of yet another embodiment of the connecting element of the present invention.

### DETAILED DESCRIPTION

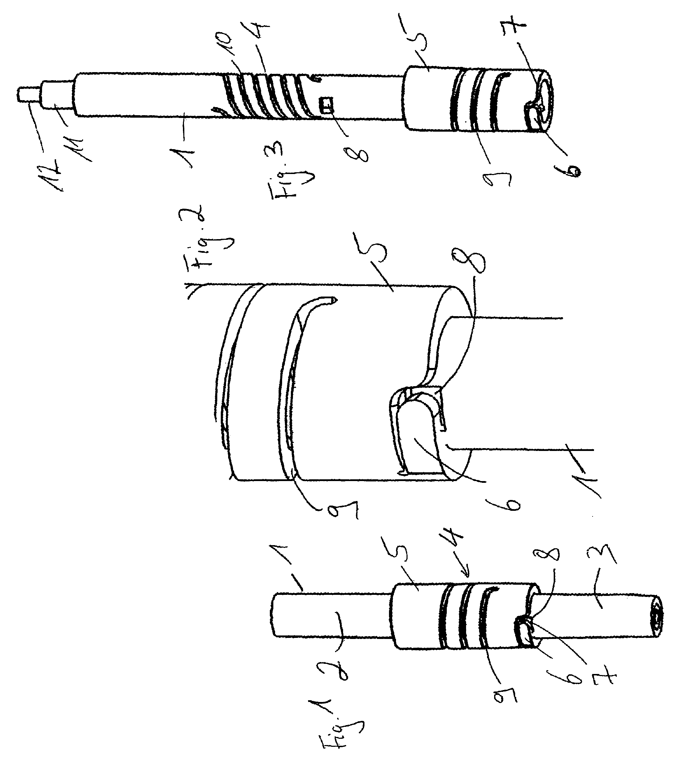

Figs. 1-3 show three-dimensional representations of a connecting element of the invention with a first rod 1 and a property adjustment element, such as a flexible element 5. The first rod has a flexible section 4 located between two connecting sections 2 and 3, the latter serving to connect the first rod 1 to the elements to be connected, such as pedicle screws for attachment to the spine.

In the embodiment shown in Fig. 1, the first rod is formed by a cylindrical tube. The connecting sections 2 and 3 form smooth sections of the first rod 1 and are suitable for arrangement in pedicle screws or especially polyaxial screws.

The force transmission between the elements to be connected, such as pedicle screws, proceeds via the first rod 1 into which the force is introduced or extracted directly via the connecting sections 2 and 3.

The flexible section 4 (see Fig. 3) extends between the connecting sections 2 and 3 of the first rod 1. In the illustration of Fig. 1, the flexible section 4 is covered by an additional flexible element 5.

Flexible element 5 is formed as a cylindrical tube, which is arranged coaxially with the first rod 1 and surrounds its flexible section 4.

Flexible element 5 has a slit 9 that completely penetrates the wall of the flexible element 5. The slit 9 has a screw shape or helical shape and imparts flexibility to flexible element 5 in the axial direction as regards extension or compression. The slit 9 also imparts flexibility as regards torsional rotation or bending about a radial rotary axis.

Flexible element 5 has, at least at one of its ends, a connecting component 6, 7 for connecting to the first rod 1. Connecting component 6, 7 is formed by a spring-loaded flap 6, which is defined by a gap extending into the flexible element 5 from the end of the flexible element 5. At a free end of the spring-loaded flap 6, an inwardly projecting lug 7, also called a protuberance, projects into an opening 8 in the first rod 1 to connect the flexible element 5 to the first rod 1. A positive joint connection is thus formed, although the connection may be non-positive.

The flap 6 may be capable of flexing outwardly to allow the lug 7 to pass over the first rod 1. The spring-loaded flap 6 is formed such that the projecting lug 7 enters into the opening 8, also called a recess, like a spring. In the unloaded state, the spring-loaded flap 6 is aligned with the cylindrical-tube body of flexible element 5. Alternatively, the flap 6 is not formed as a spring-loaded flap, but rather is formed as a bending flap. After positioning the flexible element 5 over the flexible section 4 of the first rod 1, the projecting lug 7 of the bending flap 6 may be forced into the opening 8 of the first rod 1 by bending of the bending flap 6.

Fig. 2 shows a magnified representation of the connection between the flexible element 5 and the first rod 1, with the connecting component 6, 7 engaging the opening 8, as described above.

In Figs. 1 and 2, only a single connecting location is provided at the lower end of the flexible element 5. This leads to the flexible element 5, despite axial compression or extension of the first rod 1, not being loaded in the axial direction since the first rod 1 can move freely in a through-hole of the tubular flexible element 5 and only one connection is present between the flexible element 5 and the first rod 1. That is, the single connection is formed by the flap 6 and the projecting lug 7 arranged thereon, and the engagement of the projecting lug 7 in the opening 8.

Alternatively, at the two opposite ends of the flexible element 5, corresponding connections may be provided such that, in the event of axial loading of the first rod 1, the flexible element 5 is also loaded in the axial direction and is extended or compressed according to the axial load.

If only one connection is provided, as shown in Figs. 1 and 2, the flexible element essentially helps to facilitate or generally influence bending about radial axes, i.e., rotary axes perpendicular to the longitudinal axis of the first rod 1, or to limit shearing transverse to the longitudinal axis of the rod. In the latter case, the flexible element 5 serves as a stabilizing element with regard to the shearing.

Fig. 3 shows a three-dimensional representation of the individual components of a preferred embodiment of the connecting element of the invention in accordance with Figs. 1 and 2 in an exploded state.

It may first be seen in Fig. 3 that the flexible element 5 is pushed over the first rod 1 during assembly and, provided the projecting lug 7 at the bending or spring-loaded flap 6 does not engage with the opening 8, can be displaced along the longitudinal axis.

Further, Fig. 3 shows the flexible section 4 of the first rod l, which also is formed with a spiral or screw-shaped slit 10.

As shown by comparison of Figs. 1 and 3 and of the threads of the slit 9 of the flexible element 5 and the threads of the slit 10 of the flexible section 4 of the first rod, the threads of the helix shapes of the slits 9 and 10 are designed such that they extend in opposite directions or are mirror-symmetrical to each other. Thus, shear forces transverse to the longitudinal axis of the rod can be readily accommodated by the flexible connecting element, while, at the same time, bending about rotary axes perpendicular to the longitudinal axis of the rod is possible, even if the property adjustment element influences the bending.

Further, superimposing flexible element 5 over flexible section 4 enables the flexibility or mobility, damping, extensibility and the mechanical properties in general to be adjusted. Since the flexible connecting element is modular with respect to the first rod 1 and the flexible element 5 and can be readily assembled, targeted adjustment of the mechanical properties of the flexible connecting element to the requirements of the application concerned is possible.

Furthermore, it may be seen from Fig. 3, that the first rod 1 may be formed as a cylindrical tube. A second rod 11 and a third rod 12 are provided within the first rod 1, with the rod 11 formed as a cylindrical tube in which the cylindrical solid rod 12 is arranged. Rods 11 and 12 are formed preferably to be flexible or elastic or generally deformable, such that the flexible connecting element overall has a flexible and deformable structure. The second rod 11 and the third rod 12 may preferably be made of a plastic material that is inherently sufficiently elastic or flexible, or generally reversibly deformable. Alternatively, the rods 11 and 12 may be made of a metallic material, which has corresponding elastic properties, or especially pseudo-elastic or super-elastic properties, such as nitinol.

Also, the first rod 1 and the flexible element 5 may be made of elastic or flexible or generally reversibly deformable materials, for which the pseudo-elastic or super-elastic material nitinol is especially suitable, which is an alloy of nickel and titanium.

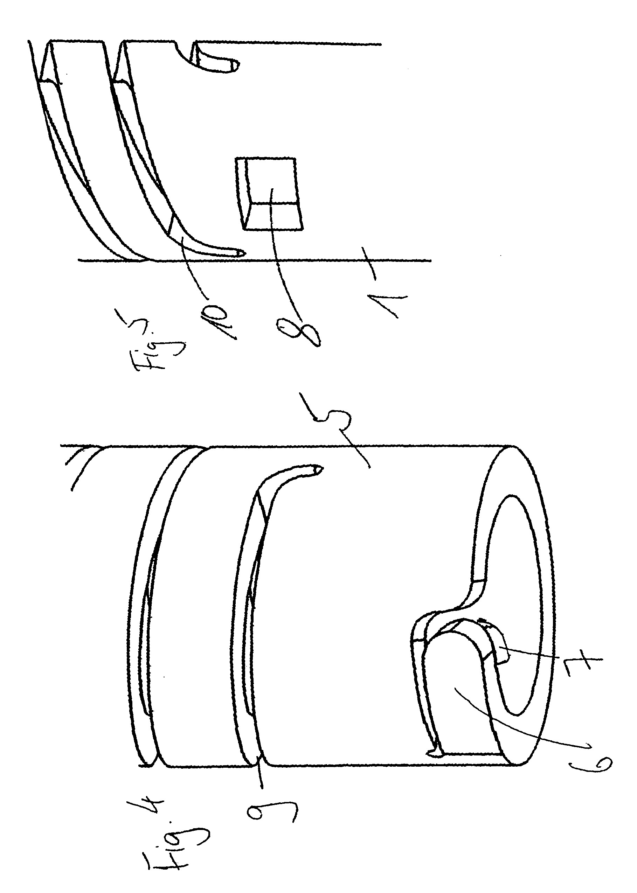

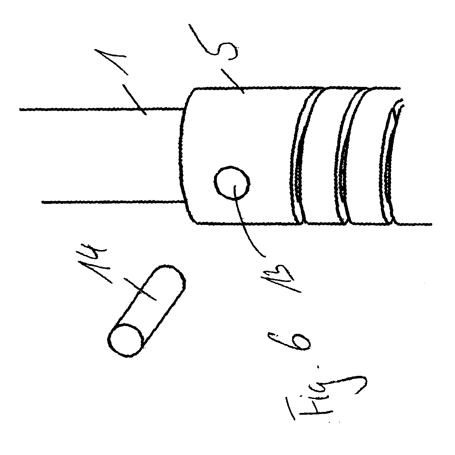

Figs. 4 to 6 show two different possibilities for connecting the flexible element 5 to the first rod 1. While Figs. 4 and 5 detail the positive connection as represented in the embodiment of Figs. 1 to 3, Fig. 6 shows a further alternative of a positive connection effected by connecting pins 14. To form this connection, corresponding pin 14 is pushed into two aligned openings of the flexible element 5 and the first rod 1, such that the flexible element 5 is immobilized relative to the first rod 1. Fig. 6 shows such an opening 13 of the flexible element 5. The underlying opening of the first rod 1 cannot be seen in the representation in Fig. 6.

Aside from the illustrated connecting techniques for the connection between flexible element 5 and first rod 1, other suitable connecting techniques are possible, such as screw connections or the like. In a screw connection, for example, the embodiment of Fig. 6 would include threads on the pin 14 and the corresponding threads on both the opening 13 of the flexible element 5 and the opening of the first rod 1.

Generally, any connecting technique, including non-positive frictional and material connecting techniques may be used to connect the flexible element 5 and the first rod 1 of the connecting element of the invention.

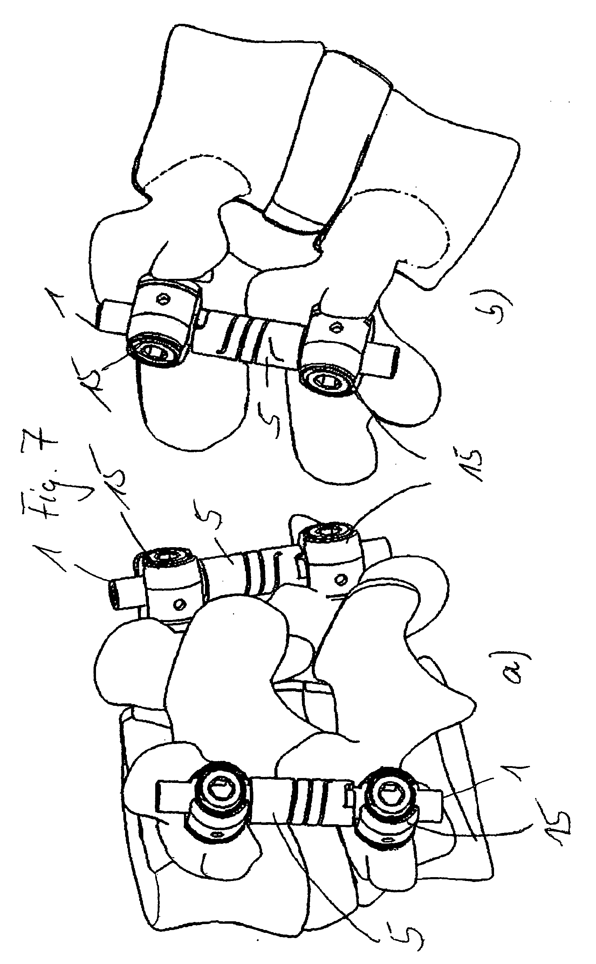

Figures 7a and 7b show the application of stabilization systems of the invention comprising of polyaxial-pedicle screws 15, which are screwed into the vertebrae of the spine, and of flexible connecting elements. In the embodiments shown, between each of two polyaxial screws 15 is provided a flexible connecting element with a flexible section in the middle, around which an outer flexible element is arranged. The flexible section of the flexible connecting element or the element arranged outside the cross-section of the flexible section may abut at the heads of the pedicle screws or may be spaced apart, preferably in the order of magnitude of tenths of a millimetre to several millimeters. The flexible connecting element corresponds to the embodiment as represented in Figs. 1 to 3.

The connecting sections 2 and 3 of the first rod 1 are received into screw heads corresponding to the polaxial-pedicle screws 15 and fixed in place. The use of the polyaxial-pedicle screws makes it possible, to a certain extent, to freely orient the longitudinal axis of the first rod 1 of the flexible connecting element of the invention relative to the longitudinal axis of the pedicle screws 15. All types of pedicle screws having the corresponding properties may serve as the polyaxial screws. Although polyaxial screws are preferred, additionally all other pedicle screws or generally known anchoring elements may conceivably be used for attachment to the connecting sections 2 and 3 of the flexible connecting element.

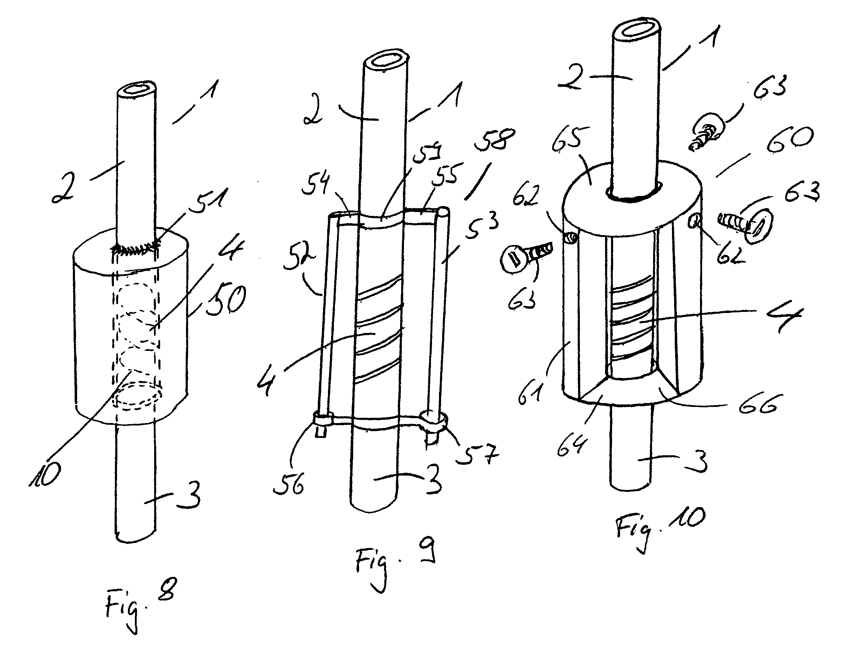

Figures 8 to 10 show alternative embodiments of the present invention. In the embodiment shown in Fig. 8 a stabilization element 50 is disposed at the first rod 1 instead of the flexible element 5 shown in Fig. 1. The first rod 1 is identical to that one of the embodiment of Fig. 1. Accordingly the numerals with respect to the first rod 1 are identical to that of Fig. 1.

Stabilization element 50 is formed as a cylindrical-tube in which the first rod 1 is inserted such that the flexible section 4 of the first rod 1 is covered by the stabilization element 50. In order to allow limited bending of the first rod 1, the inner diameter of the through-hole of stabilization element 50 is greater than the outer diameter of the first rod l. The device in Fig. 8 improves stabilization because the stabilization element improves shearing properties and limits bending of the first rod.

The stabilization element 50 is fixed to the first rod 1 by a circumferential joint weld 51 which surrounds the first rod 1 in the shape of a ring. Since first rod 1 and stabilization element 50 are only connected at a single connecting location at the upper end of stabilization element 50, axial compression or elongation of the first rod 1 is possible

Fig. 9 shows a further embodiment of a stabilization element 58 comprising a strip 52 and a cylindrical bar 53. The upper ends of the strip 52 and the bar 53 are immovably fixed to the first rod 1 by ligaments 54 and 55 firmly connected to the first rod 1. The lower ends of the strip 52 and the bar 53 are guided by rings 56 and 57, respectively, which are also firmly attached to the first rod 1. Since the rings 56 and 57 are not firmly fixed to the strip 52 or the bar 53, respectively, the flexible connecting element can be compressed in the axial direction without interaction of the strip 52 or the bar 53. However, the stabilization element comprising the strip 52 and the bar 53 provides stabilization with respect to bending of the first rod 1 dependent on the amount of gap between the strip/bar and the rings. Although, the stabilization element 58 of Fig. 9 comprises a strip 52 and a bar 53 it is also possible to only comprise either strips or bars.

Fig. 10 shows a further embodiment of the flexible connecting element of the present invention. The flexible connecting element of Fig. 10 also comprises a first rod 1 being identical to the embodiments of Fig. 1, 8 and 9. Accordingly identical numerals are used for designating corresponding components.

The flexible element 60 shown in Fig. 10 is formed by a partial cylinder 61 which comprises openings 66 in the jacket wall extending from an upper ring plate 65 to a lower ring plate 64. The partial cylinder 61 is fixed to the first rod 1 by screw connections. The screws 63 of the screw connections are engaged within holes 62 of the partial cylinder 61 so as to allow the threaded shafts of the screws 63 to cooperate with threaded holes of the first rod 1 (not shown). Thus, the connection of the flexible element 60 with the first rod 1 is established in the upper part of the partial cylinder 61. Since the flexible element 60 and the first rod 1 are not connected at the lower end opposite to the connecting area, the first rod 1 can be compressed or elongated independently in the axial direction. Due to the openings 66 in the jacket wall, the design of the element 60 is such that an inherent flexibility, e. g. with respect to bending forces, is given, similar to the flexible element 5. Contrary to that, the stabilization elements 50 and 58 of the embodiments shown in Figs. 8 and 9 have a design predominantly providing stability.

From the embodiments shown in Figs. 1, 8, 9 and 10 it also becomes evident that different connecting techniques for connecting the flexible element or stabilization element to the first rod 1 can be used.

While in the embodiment shown in Fig. 1 the flexible element 5 is connected to the first rod 1 by positive locking, the stabilization element 50 of the embodiment shown in Fig. 8 is connected to the first rod 1 by a material connection in the form of a welding.

Further, the ligaments 55 and 54 of the embodiment shown in Fig. 9 are attached to the first rod 1 by a ring 59 surrounding the first rod 1 circumferentially in a non-positive frictional manner.

In addition, the screw connections 63 of the embodiment shown in Fig. 10 can be seen as a combination of positive and non-positive connecting techniques, wherein the tightening forces of the screws provide the non-positive part while the engagement of the screw shafts in the threaded holes can be seen as form fit.

## Claims

1. Flexible connecting element for implantation into human or animal body comprising:
a first rod (1) having at least one flexible section and at least two connecting sections with the flexible section (4) being located between two connecting sections (2,3), the two connecting sections being adapted for attachment to devices to be secured to the human or animal body and the flexible section having greater flexibility than each of the connecting section;
and
a property adjustment element (5) located outside the cross-section of the first rod and extending along at least a part of the flexible section (4) of the first rod
**characterized by**
the property adjustment element being connected to the first rod at a first location such that the property adjustment element is axially immovable relative to the first rod at the first location.

2. The flexible connecting element of claim 1, wherein the property adjustment element (5) is a flexible element or a stabilization element formed as a rod, strip, partial cylinder or as a cylindrical-tubular body.

3. The flexible connecting element of claim 1 or 2, wherein the property adjustment element is a flexible elements, the flexibility of which is achieved by design and/or flexible material.

4. The flexible connecting element of any of the previous claims, wherein the property adjustment element has a cross-section reduction, a wall thickness reduction, material recesses, flexible reformable material or reversibly flexible deformable material.

5. The flexible connecting element of any of the previous claims, wherein the property adjustment element has a cylindrical-tubular body having at least one slit or grooved material recess (9) for obtaining mobility in axial extension or compression, torsional rotation or bending about a radial axis.

6. The flexible connecting element of claim 5, wherein the slit or grooved material recess (9) is provided spirally or helically about a longitudinal axis of the property adjustment element.

7. The flexible connecting element of any of the previous claims, wherein the property adjustment element is provided only over the flexible section (4) of the first rod (1).

8. The flexible connecting element of any of the previous claims, wherein the property adjustment element is connected to the first rod at one end of the property adjustment element and at one end of the flexible section of the first rod.

9. The flexible connecting element of any of the previous claims, wherein the property adjustment element is connected to the first rod by a positive connection or a non-positive connection.

10. The flexible connecting element of any of the previous claims, wherein the property adjustment element has at least one inwardly projecting protuberance that engages with a recess of the first rod.

11. The flexible connecting element of claim 10, wherein the protuberance is located non a spring-loaded flap or a bending flap.

12. The flexible connecting element of any of the previous claims, wherein one or more pins (14) or screws are accommodated in corresponding openings (13) of the property adjustment element and the first rod for connecting the property adjustment element to the first rod.

13. The flexible connecting element of any of the previous claims, wherein a single connection between the property adjustment element and the first rod is provided, especially in a plane perpendicular to a longitudinal axis of the property adjustment element.

14. The flexible connecting element of any of the previous claims, wherein the first rod comprises a cylindrical solid body or a cylindrical-tubular body.

15. The flexible connecting element of any of the previous claims, wherein the flexible section (4) of the first rod has a cross section reduction, a wall thickness reduction, material recesses or flexible material.

16. The flexible connecting element of any of the previous claims, wherein the flexibility of the flexible section (4) of the first rod is achieved by design and/or flexible material.

17. The flexible connecting element of any of the previous claims, wherein the flexible section (4) of the first rod has at least one helical or screw type slit or grooved material recess for obtaining mobility in axial extension or compression torsional rotation or bending about a radial axis.

18. The flexible connecting element of claims 5 and 17, wherein the slit or grooved material recess of the flexible element is provided spirally or helically about a longitudinal axis of the property adjustment element and windings of the slit or grooved material recess of the first rod run in opposing directions and/or are mirror symmetrical to the windings of the property adjustment element.

19. The flexible connecting element of any of the previous claims, further comprising at least a second rod (11, 12), especially coaxial to and provided within the first rod.

20. The flexible connecting element of claim 19, wherein the second rod has a cylindrical solid body or a cylindrical-tubular body.

21. The flexible connecting element of claim 20, wherein the second rod is connected to the first rod at only one location or several points in a single or small section, especially an end section.

22. The flexible connecting element of any of the previous claims, wherein at least one of the first rod, a second rod and the property adjustment element is made of a flexible material, especially a biocompatible plastic or metal, preferably a super- or pseudoelastic material, in particular nitinol.

23. The flexible connecting element of any of the previous claims, wherein the first rod and the property adjustment element are made of nitinol and a second rod is made of plastic, especially polyethylene, polytetrafluoroethylene, or polyethylene terephthalate.

24. The flexible connecting element of any of the previous claims, wherein the property adjustment element, the first rod and/or a second rod are each formed as one piece.

25. A stabilization system for the spine comprising:
a flexible connecting element of any of the previous claims and two anchoring elements being connectable respectively to the two connecting sections of the flexible connecting element and to a vertebrae of the spine.

26. The stabilization system of claim 25, wherein the anchoring elements are pedicle screws, especially polyaxial screws.

27. A modular system of one or more first rods and one or several property adjustment elements of the flexible connecting element according to one of the claims 1 to 24 and one or more anchoring elements.

## Patentansprüche

1. Flexibles Verbindungselement für die Implantation in den menschlichen oder tierischen Körper, das umfasst:
einen ersten Stab (1), der mindestens einen flexiblen Bereich (4) und mindestens zwei Verbindungsbereiche aufweist, wobei der flexible Bereich zwischen den zwei Verbindungsbereichen (2,3) angeordnet ist, wobei die zwei Verbindungsbereiche für das Anbringen an Vorrichtungen ausgelegt sind, die im menschlichen oder tierischen Körper befestigt werden, und wobei der flexible Bereich eine größere Flexibilität als jeder der Verbindungsbereiche aufweist,
und
ein Eigenschaftsjustierelement (5), das außerhalb des Querschnitts des ersten Stabes angeordnet ist und das sich entlang von wenigstens einem Teil des flexiblen Bereichs (4) des ersten Stabes erstreckt,
**dadurch gekennzeichnet, dass**
das Eigenschaftsjustierelement mit dem ersten Stab an einer ersten Stelle so verbunden ist, dass das Eigenschaftsjustierelement axial unbeweglich relativ zu dem ersten Stab an der ersten Stelle ist.

2. Flexibles Verbindungselement nach Anspruch 1, bei welchem das Eigenschaftsjustierelement (5) ein flexibles Element oder ein Stabilisierungselement ist, das als Stab, Streifen, Teilzylinder oder als zylinderrohrartiger Körper ausgebildet ist.

3. Flexibles Verbindungselement nach Anspruch 1 oder 2, bei welchem das Eigenschaftsjustierelement ein flexibles Element ist, dessen Flexibilität durch konstruktive Maßnahmen und/oder flexibles Material erreicht wird.

4. Flexibles Verbindungselement nach einem der vorhergehenden Ansprüche, bei welchem das Eigenschaftsjustierelement eine Querschnittsverringerung, eine Wanddickenverringerung, Materialaussparungen, flexibles, verformbares Material oder reversibel flexibles, verformbares Material umfasst.

5. Flexibles Verbindungselement nach einem der vorhergehenden Ansprüche, bei welchem das Eigenschaftsjustierelement einen zylinderrohrartigen Körper aufweist, der zur Erzielung einer Beweglichkeit in Bezug auf eine axiale Streckung oder Stauchung, torsiale Drehung oder einer Biegung um eine radiale Achse mindestens eine schlitz- oder nutförmige Materialaussparung (9) aufweist.

6. Flexibles Verbindungselement nach Anspruch 5, bei welchem die schlitz- oder nutenförmige Materialaussparung (9) spiral- oder wendelförmig um eine Längsachse des Eigenschaftsjustierelements vorgesehen ist.

7. Flexibles Verbindungselement nach einem der vorhergehenden Ansprüche, bei welchem das Eigenschaftsjustierelement nur über dem flexiblen Bereich (4) des ersten Stabes (1) vorgesehen ist.

8. Flexibles Verbindungselement nach einem der vorhergehenden Ansprüche, bei welchem das Eigenschaftsjustierelement mit dem ersten Stab an einem Ende des Eigenschaftsjustierelements und an einem Ende des flexiblen Bereichs des ersten Stabes verbunden ist

9. Flexibles Verbindungselement nach einem der vorhergehenden Ansprüche, bei welchem das Eigenschaftsjustierelement durch form- oder kraftschlüssige Verbindung mit dem ersten Stab (1) verbunden ist.

10. Flexibles Verbindungselement nach einem der vorhergehenden Ansprüche, bei welchem das Eigenschaftsjustierelement wenigstens einen nach innen gerichteten "Vorsprung umfasst, der in Eingriff mit einer Ausnehmung des ersten Stabes steht.

11. Flexibles Verbindungselement nach Anspruch 10, bei welchem der Vorsprung an einer durch eine Feder vorgespannten Lasche oder einer Biegelasche angeordnet ist.

12. Flexibles Verbindungselement nach einem der vorhergehenden Ansprüche, bei welchem ein oder mehrere Stifte (14) oder Schrauben zur Verbindung von Eigenschaftsjustierelement und erstem Stab in entsprechenden Öffnungen (13) des Eigenschaftsjustierelements und des ersten Stabes aufgenommen sind.

13. Flexibles Verbindungselement nach einem der vorhergehenden Ansprüche, bei welchem nur eine einzige Verbindung, insbesondere in einer Ebene senkrecht zur Längsachse des Eigenschaftsjustierelements, zwischen Eigenschaftsjustierelement und erstem Stab vorgesehen ist.

14. Flexibles Verbindungselement nach einem der vorhergehenden Ansprüche, bei welchem der erste Stab einen zylinderartiger Vollkörper oder einen zylinderrohrartiger Körper umfasst.

15. Flexibles Verbindungselement nach einem der vorhergehenden Ansprüche, bei welchem der flexible Bereich (4) des ersten Stabes eine Querschnittsverringerung, eine Wandstärkenreduzierung, Materialaussparungen oder flexibles Material aufweist.

16. Flexibles Verbindungselement nach einem der vorhergehenden Ansprüche, bei welchem die Flexibilität des flexiblen Bereichs (4) des ersten Stabes durch konstruktive Maßnahmen und/oder durch flexibles Material erreicht wird.

17. Flexibles Verbindungselement nach einem der vorhergehenden Ansprüche, bei welchem der flexible Bereich (4) des ersten Stabes mindestens eine wendel- oder schraubenförmige, schlitz- und/oder nutförmige Materialaussparung zur Erzielung einer Beweglichkeit in Bezug auf eine axiale Streckung oder Stauchung, torsiale Drehung oder auf eine Biegung um eine radiale Achse aufweist.

18. Flexibles Verbindungselement nach den Ansprüchen 5 und 17, bei welchem die schlitz- oder nutenförmige Materialaussparung (9) spiral- oder wendelförmig um eine Längsachse des Eigenschaftsjustierelements vorgesehen ist und bei welchem Windungen der schlitz- oder nutförmigen Materialaussparungen des ersten Stabes gegenläufig und/oder spiegelsymmetrisch zu den Windungen des Eigenschaftsjustierelements sind.

19. Flexibles Verbindungselement nach einem der vorhergehenden Ansprüche, das ferner mindestens ein zweiter Stab (11, 12) umfasst, der insbesondere koaxial zu und innerhalb des ersten Stabes vorgesehen ist.

20. Flexibles Verbindungselement nach Anspruch 19, bei welchem der zweite Stab einen zylindrischen Vollkörper oder einen zylinderrohrartigen Körper aufweist.

21. Flexibles Verbindungselement nach Anspruch 20, bei welchem der zweite Stab mit dem ersten Stab nur an einer Stelle oder an mehreren Punkten in einem oder einem kleinen Bereich, insbesondere einem Endbereich, verbunden ist.

22. Flexibles Verbindungselement nach einem der vorhergehenden Ansprüche, bei welchem mindestens der erste Stab, ein zweiter Stab oder das Eigenschaftsjustierelement aus einem flexiblen Material, insbesondere einem biokompatiblen Kunststoff oder Metall gebildet sind, insbesondere aus einem super- oder pseudoelastischen Material, vorzugsweise Nitinol.

23. Flexibles Verbindungselement nach einem der vorhergehenden Ansprüche, bei welchem der erste Stab und das Eigenschaftsjustierelement aus Nitinol und ein zweiter Stab aus Kunststoff, insbesondere Polyethylen, Polytetrafluorethylen oder Polyethylenterephthalat, gebildet sind.

24. Flexibles Verbindungselement nach einem der vorhergehenden Ansprüche, bei welchem das Eigenschaftsjustierelement, der erste Stab und/oder ein zweiter Stab jeweils einstückig gebildet sind.

25. Stabilisierungssystem für die Wirbelsäule, das umfasst:
ein flexibles Verbindungselement nach einem der vorhergehenden Ansprüche und zwei Verankerungselemente, die jeweils an den zwei Verbindungsbereichen des flexiblen Verbindungselements und in einem Wirbelkörper der Wirbelsäule befestigbar sind.

26. Stabilisierungssystem nach Anspruch 25, bei welchem die Verankerungselemente Pedikelschrauben, insbesondere Polyaxialschrauben sind.

27. Modulares System mit einem oder mehreren ersten Stäben und einem oder mehreren Eigenschaftsjustierelementen des flexiblen Verbindungselements gemäß einem der Ansprüche 1 bis 24 und einem oder mehreren Verankerungselementen.

## Revendications

1. Elément de connexion flexible pour implanter dans le corps d'un être humain ou d'un animal comprenant :
une première tige (1) ayant au moins une section flexible et au moins deux sections de connexion avec la section flexible (4) étant située entre deux sections de connexion (2, 3), les deux sections de connexion étant adaptées pour être attachées à des dispositifs à fixer sur le corps d'un être humain ou d'un animal et la section flexible ayant une plus grande flexibilité que chacune des sections de connexion
et
un élément d'ajustage de propriété (5) situé à l'extérieur de la section transversale de la première tige et qui s'étend le long d'au moins une partie de la section flexible (4) de la première tige
**caractérisé par**
l'élément d'ajustage de propriété qui est connecté à la première tige à un premier endroit de telle manière que l'élément d'ajustage de propriété est immobile axialement par rapport à la première tige au premier endroit.

2. Elément de connexion flexible selon la revendication 1 dans lequel l'élément d'ajustage de propriété (5) est un élément flexible ou un élément de stabilisation formé comme une tige, une bande, un cylindre partiel ou comme un corps tubulaire cylindrique.

3. Elément de connexion flexible selon la revendication 1 ou 2 dans lequel l'élément d'ajustage de propriété est un élément flexible, la flexibilité duquel est obtenue par sa conception et/ou un matériau flexible.

4. Elément de connexion flexible selon l'une des revendications précédentes dans lequel l'élément d'ajustage de propriété a une réduction de section transversale, une réduction d'épaisseur de paroi, des évidements de matériau, du matériau déformable flexible ou du matériau déformation flexible de manière réversible.

5. Elément de connexion flexible selon l'une quelconque des revendications précédentes dans lequel l'élément d'ajustage de propriété a un corps tubulaire cylindrique ayant au moins une fente ou un évidement de matériau rainuré (9) pour obtenir de la mobilité dans l'extension axiale ou la compression, une rotation en torsion ou une flexion autour d'un axe radial.

6. Elément de connexion flexible selon la revendication 5 dans lequel la fente ou l'évidement de matériau rainuré (9) est prévu en spirale ou hélicoïdal autour d'un axe longitudinal de l'élément d'ajustage de propriété.

7. Elément de connexion flexible selon l'une quelconque des revendications précédentes dans lequel l'élément d'ajustage de propriété est prévu seulement sur la section flexible (4) de la première tige (1).

8. Elément de connexion flexible selon l'une quelconque des revendications précédentes dans lequel l'élément d'ajustage de propriété est relié à la première tige à une extrémité de l'élément d'ajustage de propriété et à une extrémité de la section flexible de la première tige.

9. Elément de connexion flexible selon l'une quelconque des revendications précédentes dans lequel l'élément d'ajustage de propriété est relié à la première tige par une connexion positive ou une connexion non positive.

10. Elément de connexion flexible selon l'une quelconque des revendications précédentes dans lequel l'élément d'ajustage de propriété a au moins une protubérance qui fait saillie vers l'intérieur qui se met en prise avec un évidement de la première tige.

11. Elément de connexion flexible selon la revendication 10 dans lequel la protubérance est située sur une patte chargée par ressort ou sur une patte de flexion.

12. Elément de connexion flexible selon l'une quelconque des revendications précédentes dans lequel une ou plusieurs chevilles (14) ou vis sont logées dans des ouvertures correspondantes (13) de l'élément d'ajustage de propriété et la première tige pour relier l'élément d'ajustage de propriété à la première tige.

13. Elément de connexion flexible selon l'une quelconque des revendications précédentes dans lequel une seule connexion est prévue entre l'élément d'ajustage de propriété et la première tige, en particulier dans un plan perpendiculaire à un axe longitudinal de l'élément d'ajustage de propriété.

14. Elément de connexion flexible selon l'une quelconque des revendications précédentes dans lequel la première tige comprend un corps solide cylindrique ou un corps tubulaire cylindrique.

15. Elément de connexion flexible selon l'une quelconque des revendications précédentes dans lequel la section flexible (4) de la première tige a une réduction de section transversale, une réduction d'épaisseur de paroi, des évidements de matériau ou du matériau flexible.

16. Elément de connexion flexible selon l'une quelconque des revendications précédentes dans lequel la flexibilité de la section flexible (4) de la première tige est obtenue par sa conception et/ou un matériau flexible.

17. Elément de connexion flexible selon l'une quelconque des revendications précédentes dans lequel la section flexible (4) de la première tige a une fente hélicoïdale ou de type vis ou un évidement de matériau rainuré pour obtenir de la mobilité dans l'extension axiale ou la compression, une rotation en torsion ou une flexion autour d'un axe radial.

18. Elément de connexion flexible selon les revendications 5 et 17 dans lequel la fente ou l'évidement de matériau rainuré de l'élément flexible est prévu en spirale ou hélicoïdal autour d'un axe longitudinal de l'élément d'ajustage de propriété et des enroulements de la fente ou de l'évidement de matériau rainuré de la première tige vont dans des sens opposés et/ou sont symétriques selon la symétrie miroir par rapport aux enroulements de l'élément d'ajustage de propriété.

19. Elément de connexion flexible selon l'une quelconque des revendications précédentes comprenant de plus au moins une seconde tige (11, 12), en particulier coaxiale à et prévue à l'intérieur de la première tige.

20. Elément de connexion flexible selon la revendication 19 dans lequel la seconde tige a un corps solide cylindrique ou un corps tubulaire cylindrique.

21. Elément de connexion flexible selon la revendication 20 dans lequel la seconde tige est reliée à la première tige à seulement un endroit ou en plusieurs points dans une seule ou une petite section, en particulier une section d'extrémité.

22. Elément de connexion flexible selon l'une quelconque des revendications précédentes dans lequel au moins l'un des éléments première tige, une seconde tige et l'élément d'ajustage de propriété est en un matériau flexible, en particulier un plastique ou un métal biocompatible, de préférence un matériau superélastique ou pseudoélastique, en particulier du nitinol.

23. Elément de connexion flexible selon l'une quelconque des revendications précédentes dans lequel la première tige et l'élément d'ajustage de propriété sont en nitinol et une seconde tige est en plastique, en particulier en polyéthylène, en polytétrafluoroéthylène ou en téréphtalate de polyéthylène.

24. Elément de connexion flexible selon l'une quelconque des revendications précédentes dans lequel l'élément d'ajustage de propriété, la première tige et/ou une seconde tige sont formés chacun en une partie.

25. Système de stabilisation pour la colonne vertébrale comprenant :
un élément de connexion flexible selon l'une quelconque des revendications précédentes et deux éléments d'ancrage pouvant être reliés respectivement aux deux sections de connexion de l'élément de connexion flexible et à une vertèbre de la colonne vertébrale.

26. Système de stabilisation selon la revendication 25 dans lequel les éléments d'ancrage sont des vis pédiculaires, en particulier des vis polyaxiales.

27. Système modulaire d'une ou de plusieurs premières tiges et d'un ou de plusieurs éléments d'ajustage de propriété de l'élément de connexion flexible selon l'une des revendications 1 à 24 et d'un ou de plusieurs éléments d'ancrage.
